# EUROPÄISCHE PATENTANMELDUNG

(11) **EP 2 450 313 A1**
(43) Veröffentlichungstag der Anmeldung: **09.05.2012**
(21) Anmeldenummer: 10014404.7
(22) Anmeldetag: 09.11.2010
(51) Int. Cl.: C02F 1/461, C02F 1/68, A23L 2/44, A61L 2/03

(54) **Verfahren zur Erzeugung einer elektrochemisch aktvierten Lösung durch Elektrolyse**

(71) Anmelder: Caliopa AG, 6314 Unterägeri (CH)
(72) Erfinder: Mathé, Hans-Georg, 6330 Cham (CH)
(74) Vertreter: Tergau & Walkenhorst

(57) **Zusammenfassung**

Ein Verfahren zur Erzeugung einer elektrochemisch aktivierten Lösung durch Elektrolyse von solehaltigem Wasser soll eine besonders hohe Lagerbeständigkeit des Anolyten gewährleisten. Dazu wird dem Anolyten erfindungsgemäß ein Carbonat von Natrium als Stabilisator zugesetzt.

## Beschreibung

Die Erfindung betrifft ein Verfahren zur Erzeugung einer elektrochemisch aktivierten Lösung durch Elektrolyse von solehaltigem Wasser.

Konzepte zur Erzeugung einer elektrochemisch aktivierten Lösung durch Elektrolyse von solehaltigem Wasser, bei denen eine oder mehrerer Elektrolysezellen zum Einsatz kommen, sind aus einer Vielzahl von Dokumenten, beispielsweise aus der DE 30 003 131 A1, aus der US 4,056,452, aus der EP 1 728 768 A1 oder aus der nicht vorveröffentlichten europäischen Patentanmeldung Nr. 10 003 555.9-2104 bekannt. In derartigen Anlagen wird zur Erzeugung der elektrochemisch aktivierten Salzlösung durch Elektrolyse ein mit einer Kochsalzlösung oder mit Sole beaufschlagter Wasserstrom der Elektrolysevorrichtung zugeführt und dort elektrolytisch zersetzt. Dadurch wird eine elektrochemisch aktivierte wässrige Salzlösung erhalten, die einen vergleichsweise hohen Gehalt an freiem Chlor und ein vergleichsweise hohes Redox-Potential aufweist. Die dabei erhältliche elektrochemisch aktivierte Salzlösung ist besonders günstig als Desinfektionsmittel, beispielsweise zur Entkeimung von Wasser und/oder wässrigen Lösungen, nutzbar.

Bei der Herstellung der elektrochemisch aktivierten Salzlösung auf die genannte Weise wird üblicherweise eine besonders hohe Wirksamkeit oder Biozidität der hergestellten Substanz, üblicherweise charakterisiert durch einen besonders hohen Gehalt an freiem Chlor und/oder ein angemessen hohes Redox-Potential, angestrebt. Zudem ist gerade im Hinblick auf mögliche Anwendungen derartiger Substanzen als Desinfektionsmittel oder auch als Zusatz-, Träger- oder sogar Wirkstoff in medizinischen oder therapeutischen Präparaten wünschenswert, dass die hohe Wirksamkeit gerade im Hinblick auf eine besonders gute Lagerbeständigkeit auch nach vergleichsweise langer Lagerung von beispielsweise mehr als einem Jahr nahezu unverändert oder mit nur geringfügigen Veränderungen aufrecht erhalten bleiben soll. Ein weiterer wichtiger Kennwert für die Charakterisierung der auf die genannte Weise erhältlichen elektrochemisch aktivierten Salzlösung ist deren pH-Wert, der die Kompatibilität und Vereinbarkeit mit anderen chemischen Substanzen oder Wirkstoffen und auch die Verwendbarkeit der elektrochemisch aktivierten Salzlösungen in verschiedenen Umgebungsbedingungen und der Gleichen wesentlich mitbestimmt.

Bei der Herstellung von elektrochemisch aktivierten Salzlösungen der genannten Art durch Elektrolyse besteht somit allgemein das Bestreben, bei hoher Lagerbeständigkeit eine besonders hohe bakteriozide oder antibakterielle Wirkung, charakterisiert durch einen hohen Gehalt an freiem Chlor, zu gewährleisten, wobei der pH-Wert möglichst eine gute Vereinbarkeit mit anderen Substanzen oder Wirkstoffen erlauben oder sogar in der Art eines freien Parameters unabhängig von den anderen genannten Parametern einstellbar gehalten werden sollte. Gerade im Hinblick auf diese Parameter und auf ihre Vereinbarkeit miteinander sind die bekannten Konzepte zur Herstellung der elektrochemisch aktivierten Salzlösung jedoch nur begrenzt einsetzbar.

Der Erfindung liegt daher die Aufgabe zugrunde, ein Verfahren zur Erzeugung einer elektrochemisch aktivierten Lösung der oben genannten Art anzugeben, die die genannten Anforderungen in besonders weitgehendem Maße erfüllt und dabei eine besonders hohe Lagerbeständigkeit aufweist.

Diese Aufgabe wird erfindungsgemäß gelöst, indem dem Anolyten in der Art eines Stabilisators ein Carbonat, vorzugsweise ein Carbonat von Natrium, insbesondere Natriumcarbonat oder Natriumbicarbonat, zugesetzt wird.

Die Erfindung geht dabei von der Überlegung aus, dass unter Rückgriff auf das aus der nicht vorveröffentlichten europäischen Patentanmeldung Nr. 10 003 555.9-2104, deren Offenbarung ausdrücklich mit einbezogen wird ("incorporation by reference"), bekannte Konzept sowie unter Nutzung der dort beschriebenen Anlage eine elektrochemisch aktivierten Salzlösung mit besonders hoher bakteriozider oder antibakterieller Wirkung bereitgestellt werden kann, die im wesentlichen die genannten günstigen Eigenschaften aufweist. Um gerade für eine derartige Salzlösung aber auch noch eine besonders gute und langfristige Lagerfähigkeit, selbst unter vergleichsweise ungünstigen Lagerbedingungen, sicherstellen zu können, sollte eine geeignete Stabilisierung des hergestellten Anolyten erfolgen. Wie sich überraschender Weise herausgestellt hat, kann eine derartige Stabilisierung durch Zumischung eines geeigneten Stabilisator-Materials zum Anolyten vorgenommen werden. Als Stabilisator ist hierbei ein Carbonat, insbesondere Natriumcarbonat oder Natriumbicarbonat, vorgesehen.

Eine besonders günstige Stabilisierungswirkung ist dabei erreichbar, indem der Stabilisator vorteilhafterweise in geeigneter Dosierung dem Anolyten zugesetzt wird. Vorzugsweise wird dem Anolyten dabei das Carbonat, insbesondere das Natriumcarbonat oder das Natriumbicarbonat, in einer Menge von zwischen 200 mg/l und 1200 mg/l, besonders bevorzugt in einer Menge von 600 mg/l bis 800 mg/l, zugesetzt.

In weiterer vorteilhafter Ausgestaltung wird bei der Herstellung der elektrochemisch aktivierten Salzlösung eine Anlage der in der nicht vorveröffentlichten europäischen Patentanmeldung Nr. 10 003 555.9-2104 genannten Art, also insbesondere umfassend eine Mehrzahl von mit ihren Anodenräumen medienseitig in Reihe geschalteten Elektrolysezellen, verwendet, wobei der Elektrolyt den Kathodenräumen der Elektrolysezellen parallel und den Anodenräumen der Elektrolysezellen in Reihenschaltung zugeführt wird. Wie sich nämlich überraschenderweise herausgestellt hat, kann gerade auf diese Weise im Anolyten nach dem Durchlaufen von mehreren, insbesondere von mindestens zwei, Behandlungsstufen ein besonderes hoher Gehalt von freiem Chlor von bis zu 2.000 mg/l erreicht werden, wobei der solchermaßen hergestellte Anolyt zudem auch noch eine besonders hohe Lagerbeständigkeit aufweist.

Die Zuspeisung des Stabilisators ist dabei vorzugsweise nach dem Durchlauf des Anolyten durch die in Reihe geschalteten mindestens zwei Behandlungsstufen, also in besonders vorteilhafter Ausgestaltung nach der in Strömungsrichtung des Anolyten gesehen letzten Elektrolysezelle, vorgesehen.

Besonders günstige Materialeigenschaften des Anolyts hinsichtlich des Gehalts an freiem Chlor und auch hinsichtlich der Lagerbeständigkeit sind zudem erreichbar, indem in vorteilhafter Weiterbildung die Kathodenräume der Elektrolysezellen medienabströmseitig mit dem Anodenraum der in Strömungsrichtung des Anolyten gesehen ersten Elektrolysezelle verbunden sind. Bei dieser besonders bevorzugten medienseitigen Schaltung wird der aus den Kathodenräumen der Elektrolysezellen abströmende Elektrolyt - vorzugsweise ein daraus geeignet abgezweigter Teilstrom - dem Anodenraum der in Strömungsrichtung des Elektrolyten gesehen ersten Elektrolysezelle zugeführt.

Der stabilisierende Effekt des Zusatzes des Carbonats zum Anolyten ist besonders deutlich bei einer Lagerung über Raumtemperatur (in Vergleichsversuchen wurden Raumtemperatur und 40°C verglichen), aber auch bei RT vorhanden.

Dabei ist die Menge des zugesetzten Salzes ein besonders wichtiger Parameter. Bei den Grenzwerten 200g und 1.200g pro 1.000 Liter für den mit dem genannten Konzept hergestellten Anolyt ist der Effekt vergleichsweise gering; dazwischen existiert ein ausgeprägtes Maximum. Die genaue Lage des Maximums und damit der besonders bevorzugten Betriebsparameter ist von der Wirkstoffkonzentration im Anolyt abhängig, kann aber auch von weiteren Einzelheiten des Herstellungsprozesses für den Anolyten abhängen.

Beim Nachweis des Salzes im Anolyt ist zu berücksichtigen, dass sowohl Natriumals auch Carbonat-lonen im Anolyt ohnehin vorhanden sind, insbesondere durch die Kochsalzsole und die Wasserhärte (bei der Enthärtung durch lonenaustauscher werden nur die Erdalkali-lonen entfernt, nicht die Anionen). Durch die Zugabe des Salzes wird jedoch die Konzentration der Natriumionen etwa um 10% erhöht.

Eine deutlichere Erhöhung ist für die Carbonationen zu beobachten, die man beispielsweise analytisch als Kohlendioxid aus der Lösung ausgasen und volumetrisch quantifizieren könnte. Bei 15 Grad deutscher Härte (°dH) im Trinkwasser gelangen etwa 120ml detektierbares Kohlendioxid in 1 Liter Anolyt (15°dH entspricht dem Übergang von Stufe II zu III in den Härtebereichsangaben der Wasserversorger). Durch Zugabe von Natriumcarbonat der genannten Art wird etwa die gleiche Menge Kohlendioxid eingebracht, d.h. bei diesem Parameter beträgt der Unterschied zum unbehandelten Anolyt etwa 100%.

## Patentansprüche

1. Verfahren zur Erzeugung einer elektrochemisch aktivierten Lösung durch Elektrolyse von solehaltigem Wasser, bei dem dem Anolyten ein Carbonat von Natrium zugesetzt wird.

2. Verfahren nach Anspruch 1, bei dem dem Anolyten Natriumcarbonat oder Natriumbicarbonat zugesetzt wird.

3. Verfahren nach Anspruch 1 oder 2, bei dem das Carbonat dem Anolyten in einer Menge von zwischen 200mg/l und 1200 mg/l, vorzugsweise in einer Menge von etwa 600 mg/l und 800 mg/l, zugesetzt wird.

4. Verfahren nach einem der Ansprüche 1 bis 3, bei dem die Elektrolyse in einem eine Mehrzahl von Elektrolysezellen umfassenden Elektrolysemodul vorgenommen wird, bei dem jede Elektrolysezelle jeweils einen ersten Elektrodenraum und einen von diesem durch eine Membran getrennten zweiten Elektrodenraum umfasst, wobei der Elektrolyt den ersten Elektrodenräumen der Elektrolysezellen parallel und den zweiten Elektrodenräumen der Elektrolysezellen in Reihenschaltung zugeführt wird.

5. Verfahren nach Anspruch 4, bei dem der Elektrolyt den Kathodenräumen der Elektrolysezellen parallel und den Anodenräumen der Elektrolysezellen in Reihenschaltung zugeführt wird.

6. Verfahren nach Anspruch 5, bei dem aus den Kathodenräumen der Elektrolysezellen abströmender Elektrolyt dem Anodenraum der in Strömungsrichtung des Elektrolyten gesehen ersten Elektrolysezelle zugeführt wird.

7. Verfahren nach Anspruch 5 oder 6, bei dem lediglich ein Teilstrom des aus den Kathodenräumen der Elektrolysezellen abströmenden Elektrolyts dem Anodenraum der in Strömungsrichtung des Elektrolyten gesehen ersten Elektrolysezelle zugeführt wird.
